# FASCICULE DE BREVET EUROPEEN

(11) **EP 1 138 388 B1**
(45) Date de publication et mention de la délivrance du brevet: **06.04.2005**
(21) Numéro de dépôt: 01400173.9
(22) Date de dépôt: 19.01.2001
(51) Int. Cl.: B01J 31/14, B01J 31/18, B01J 31/24, B01J 31/02, C07C 2/36, C08F 10/00

(54) **Composition catalytique pour la dimérisation, la codimérisation et l'oligomérisation des oléfines**
Katalysatorzusammensetzung zur Dimerisierung, Kodimerisierung und Oligomerisierung von Olefinen
Catalytic composition for dimerisation, codimerisation and oligomerisation of olefins

(30) Priorité: 04.02.2000 FR 0001512
(43) Date de publication de la demande: 04.10.2001
(73) Titulaire: Institut Français du Pétrole, 92852 Rueil-Malmaison Cedex (FR)
(72) Inventeur: Commereuc, Dominique, 92190 Meudon (FR); Le Pennec, Dominique, 78910 Orgerus (FR); Olivier-Bourbigou, Hélène, 92500 Rueil Malmaison (FR)

(56) Documents cités:
- EP-A- 0 646 413

## Description

La présente invention concerne une composition catalytique utilisée pour là dimérisation, la codimérisation et l'oligomérisation des oléfines. Elle concerne plus particulièrement une composition résultant de la dissolution au moins partielle d'au moins un composé du nickel mélangé ou complexé avec une phosphine tertiaire ou une phosphite portant un groupe fonctionnel, dans le mélange liquide, à caractère ionique, d'au moins un halogénure d'ammonium quaternaire oulet d'au moins un halogénure de phosphonium quaternaire, d'au moins un halogénurè d'aluminium, et éventuellement d'au moins un composé organométallique d'aluminium.

Le brevet français FR-B-2 611 700 décrit l'utilisation de liquides à caractère ionique formés d'halogénures d'aluminium et d'halogénures d'ammonium quater navires comme solvants dé complexes organométalliques du nickel pour la catalyse de dimérisation des oléfines. L'utilisation de tels milieux non miscibles avec les hydrocarbures aliphatiques, en particulier avec les produits issus de la dimérisation des oléfines permet une meilleure utilisation des catatyseurs homogènes. Dans le brevet FR-B-2 659 871 est décrite une composition liquide à caractère ionique résultant de la mise en contact d'halogénures d'ammonium quartenaires et/ou d'halogénures de phosphonium quaternaires avec des dihalogénures d'alkylaluminium et éventuellement en outre un trihalogénure d'aluminium. Ce même brevet décrit l'utilisation de ces milieux comme solvants de complexes de métaux de transition, notamment des complexes du nickel ne contenant pas de liaison nickel-carbone, qui sont transformés en catalyseurs d'oligomérisation des oléfines. Dans la suite, ces milieux seront appelés « sels fondus » parce que liquides à température modéré.

Au cours de ces travaux, il a été montré que les catalyseurs de nickel les plus actifs et les plus stables sont obtenus dans des « sels fondus » constitués d'un équivalent molaire d'halogénure d'ammonium et/ou d'halogénure de phosphonium avec un équivalent et plus de trihalogénure d'aluminium, et éventuellement une quantité quelconque de dihalogénure d'alkyle aluminium. Cette formulation s'est révélée particulièrement intéressante parce que les complexes du nickel qui y sont dissous présentent une activité catalytique élevée.

en outre, la demande de brevet européen EP-A-0 646 413 décrit une composition catalytique résultant du mélange d'un halogénure d'alkylaluminium. d'un complexe du nickel (II) contenant deux molécules de phosphine tertiaire et d'un composé du nickel (II) ne contenant ni eau, ni phosphine. Les complexes du nickel (II) décrits dans ce document ne contiennent pas de phosphine tertiaire portant un groupe fonctionnel.

Il s'est avéré que dans de telles conditions et lorsque la réaction est réalisée en système semi-ouvert avec une alimentation continue en oléfine et une séparation continue des produits après décantation, une proportion faible mais non negligéable du nickel se trouve extraite dans la phase organiques.

Il a maintenant été trouvé que l'utilisation d'une phosphine tertiaire portant un groupe fonctionnel ou d'une phosphite portant un groupe fonctionnel ou d'un complexe de nickel formé avec une phosphine tertiaire ou une phosphite fonctionnalisée, soluble dans le «sel fondu », conduit à des catalyseurs dont l'activité est élevée, stable dans le temps et pour lesquels l'extraction du nickel dans les produits de la réaction est le plus possible réduite. Cela a pour conséquence de réduire la consommation de catalyseur et donc d'améliorer l'économie du procédé.

Par ailleurs, il s'est révélé que dans les conditions décrites dans le brevet français FR-B-2 611 700 « l'effet phosphine » tel que décrit par G. Wilke et al dans Ind. Eng. Chem., 1970, 62, n°12, p. 34, et dans le brevet GB-B-1 058 680, et qui traduit l'influence des substituants portés par l'atome de phosphore sur le mode d'enchaînement des molécules de propylène lors de sa dimérisation catalytique par le nickel, disparaissait rapidement au cours du temps. Ce phénomène inexpliqué a des conséquences néfastes puisqu'il ne permet pas d'obtenir les sélectivités recherchées.

Il a été montré dans le brevet FR-B-2 710 280 que l'addition d'un hydrocarbure aromatique à un « sel fondu » permet de pallier ce défaut et conduit à des catalyseurs dont l'activité est élevée et plus stable et dont la sélectivité en isomères les plus ramifiés est importante. Cependant, l'hydrocarbure aromatique est extrait en continu dans la phase organique constituée par les produits, ce qui implique qu'il soit nécessaire de le séparer et de le recycler au réacteur.

Il a maintenant été trouvé que l'utilisation d'une phosphine tertiaire portant un groupe fonctionnel ou d'un complexe de nickel complexé avec une phosphine tertiaire fonctionnalisée soluble dans le «sel fondu », conduit à des catalyseurs dont la sélectivité en isomères les plus ramifiés est importante et stable au cours du temps et dont l'activité est élevée.

L'objet de l'invention est une composition catalytique comprenant au moins un composé du nickel mélangé ou complexé avec au moins une phosphine tertiaire ou une phosphite portant un groupe fonctionnel, dissous au moins en partie dans un milieu non aqueux à caractère ionique (milieu de type « sels fondus »), résultant de la mise en contact d'au moins un halogénure d'aluminium (Produit B) avec au moins un halogénure d'ammonium quaternaire et/ou au moins un halogénure de phosphonium quaternaire (Produit A), le milieu de type « sels fondus » pouvant en outre comprendre au moins un composé organométallique d'aluminium (Produit C).

Ainsi, le milieu de type « sels fondus » dans lequel est dissous le composé de nickel mélangé ou complexé avec au moins une phosphine tertiaire portant un groupe fonctionnel ou au moins une phosphite portant un groupe fonctionnel est constitué par mélange :
a) d'au moins un halogénure, plus particulièrement chlorure et/ou bromure, d'ammonium quaternaire et/ou phosphonium quaternaire (Produit A) ;
b) d'au moins un halogénure d'aluminium (Produit B) ; et
c) éventuellement d'au moins un composé organométallique d'aluminium (Produit C).
Les halogénures d'ammonium et/ou de phosphonium quaternaires utilisables dans le cadre de l'invention (Produit A) répondent de préférence
- à l'une des formules générales NR¹R²R³R⁴X (à l'exception de NH₄X), PR¹R²R³R⁴X, R¹R²N=CR³R⁴X ou -R¹R²P=CR³R⁴X, dans lesquelles X représente Cl ou Br et R¹, R², R³ et R⁴, identiques ou différents, représentent chacun l'hydrogène ou un reste hydrocarbyle de 1 à 12 atomes de carbone par exemple des groupements alkyles, saturés ou non saturés, cycloalkyles ou aromatiques, aryles ou aralkyles, comprenant de 1 à 12 atomes de carbone, étant entendu que, de préférence, un seul des substituants R¹, R², R3 et R⁴ représente l'hydrogène;
- ou encore à l'une des formules générales : 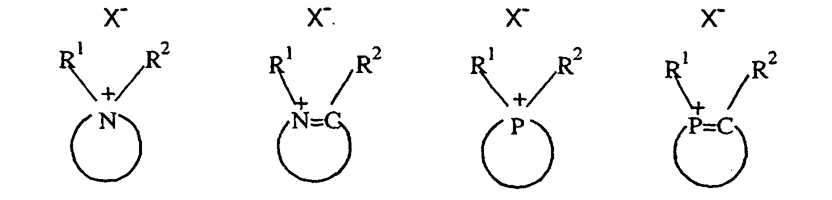
dans lesquelles les hétérocycles azotés ou phosphorés comportant 1, 2 ou 3 atomes d'azote et/ou de phosphore sont constitués de 4 à 10 atomes et X, R¹ et R² sont définis comme précédemment.

A titre d'exemples, on peut citer le chlorure de tétrabutylphosphonium, le chlorure de N-butylpyridinium, le bromure d'éthylpyridinium, le chlorure de butyl-3 méthyl-1 imidazolium, le chlorure de diéthylpyrazolium, le chlorhydrate de pyridinium, le chlorure de triméthylphénylammonium et le chlorure d'éthyl-3 méthyl-1 imidazolium. Ces sels peuvent être utilisés seuls ou en mélanges.

Les halogénures d'aluminium utilisés comme Produits B selon l'invention sont essentiellement le chlorure et le bromure d'aluminium.

Les composés organométalliques d'aluminium utilisés comme Produits C facultatifs selon l'invention, ont pour formule générale AIRₓX₃₋ₓ dans laquelle R est un reste alkyle, linéaire ou ramifié, comportant de 2 a 8 atomes de carbone, X étant le chlore ou le brome et x ayant une valeur égale à 1, 2 ou 3. A titre d'exemples, on peut utiliser le sesquichlorure d'isobutylaluminium, le sesquichlorure d'éthylaluminium, le dichloroisobutylaluminium, le dichloroéthylaluminium et le chlorodiéthylaluminium.

Les composants des « sels fondus » tels que définis ci-dessus sont en général mis en oeuvre dans des rapports molaires A:B de 1:0.5 à 1:3, de préférence de 1:1 à 1:2 ; le Produit C est mis en oeuvre dans un rapport molaire avec le Produit B au plus égal à 100:1 et de préférence de 0.005:1 à 10:1. Il est néanmoins nécessaire que les composants et leurs proportions soient tels que le mélange soit liquide à la température à laquelle se fait l'introduction du composé du nickel et la phosphine tertiaire fonctionnalisée ou de la phosphite fonctionnalisée, bien que la réaction catalytique de dimérisation puisse se faire à une température inférieure ou supérieure à la température de fusion de la composition catalytique.

Les composés du nickel utilisés dans les compositions catalytiques de l'invention sont par exemple le chlorure, le bromure, le sulfate, les carboxylates, (par exemple l'éthyl-2 hexanoate), les phénates et l'acétylacétonate. On peut également utiliser les complexes organométalliques du nickel contenant ou non des phosphines ou des phosphites. Ces complexes de nickel sont utilisés en mélange avec une phosphine tertiaire fonctionnalisée ou une phosphite fonctionnalisée. On peut également utiliser des complexes du nickel déjà complexés avec une phosphine tertiaire portant une fonction ou une phosphite portant une fonction.

Les phosphines fonctionnelles utilisées en mélange avec (ou pour complexer les composés de nickel selon l'invention) répondent aux formules générales PR'₁R'₂R'₃ et R'₁R'₂P-R'-PR'₁R'₂ dans lesquelles R'₁, R'₂ et R'₃, identiques ou différents, sont des radicaux alkyles, cycloalkyles, aryles ou aralkyles comportant de 1 à 10 atomes de carbone dont l'un au moins porte un groupe fonctionnel tel qu'une amine, une amine cyclique, un hétérocycle azoté, un ester, un acide, un alcool, un ammonium quaternaire, un phosphonium quaternaire, un sulfonium, un sulfonate ou un phosphonate et R' est un reste bivalent aliphatique de 1 à 6 atomes de carbone.

Les phosphines fonctionnelles peuvent être choisies parmi les composés contenant des substituants pyridine ou imidazole, ou leurs dérivés de quatemisation à substituants pyridinium ou imidazolium, qui répondent aux formules 1 à 7 présentées plus loin.

Comme phosphines fonctionnelles portant un substituant pyridine, on peut utiliser la dicyclopentyl-phosphinoéthyl-2-pyridine-4, de formule (1), la dicyclopentyl-phosphinoéthyl-2-pyridine-2, de formule (2), la diisobutyl-phosphinoéthyl-2-pyridine-4, de formule (1b), la diisopropyl-phosphinoéthyl-2 pyridine-4, de formule (4), et leurs dérivés de quaternisation de formule (3), dans laquelle R est un groupe alkyle comportant 1 à 10 atomes de carbone et X est un anion faiblement coordinant. A titre d'exemples d'anions faiblement coordinants, on peut citer le tétrafluoroborate, l'hexafluorophosphate, le tétrachloroaluminate, l'hexafluoroantimonate, les anions carboxylates tels que l'acétate et le trifluoroacétate, le trifluorosulfonate, ainsi que les anions N(CF₃SO₂)₂⁻ et C(CF₃SO₂)₃⁻. Les dérivés de quaternisation sont par exemple le tétrafluoroborate de dicyclopentyl-phosphinoéthyl-2 N-éthylpyridinium, de formule (3a), ou le chlorure de dicyclopentylphosphinoéthyl-2 N-éthylpyridinium, de formule (3b).

Comme phosphines fonctionnelles portant un substituant imidazole, on peut utiliser par exemple la dicyclopentyl-phosphinoéthyl-2 N-imidazole, de formule (5), la diisopropyl-phosphinoéthyl-2 N-imidazole de formule (7), la diisobutyl-phosphinoéthyl-2 N-imidazole de formule (7b) et leurs dérivés de quatemisation de formule (6), dans laquelle R est un groupe alkyle comportant 1 à 10 atomes de carbone et X est un anion faiblement coordinant (comme défini ci-dessus), tels que le tétrafluoroborate de dicyclopentyl-phosphinoéthyl-2 méthyl-1 imidazolium de formule (6a).

Les phosphites fonctionnelles utilisées en mélange avec (ou pour complexer les composés de nickel selon l'invention) répondent aux formules générales P(OR"₁)(OR"₂)(OR"₃) et (-O-R"₅ O-)P(OR"₂), dans lesquelles R"₁, R"₂, R"₃ et R"₅, identiques ou différents, sont des radicaux aryles ou aralkyles dont l'un au moins porte un groupe fonctionnel tel qu'une amine, une amine cyclique, un hétérocycle azoté, un ester, un acide, un alcool, un ammonium quaternaire, un phosphonium quaternaire, un sulfonium, un sulfonate ou un phosphonate.

Les phosphites fonctionnelles peuvent être choisies parmi les composés qui répondent aux formules 9 à 11 présentées plus loin.

On peut utiliser les phosphites représentées par la formule générale (9), (avec x de 0 à 2), dans laquelle Y⁺ peut être un cation organique tel qu'un ammonium quaternaire ou un phosphonium quaternaire de formule générale NR¹R²R³R⁴ et PR¹R²R³R⁴, où R¹, R², R³ et R⁴, identiques ou différents, représentent chacun l'hydrogène, un groupement hydrocarboné, aliphatique (saturé ou insaturé) ou aromatique, comprenant de 1 à 12 atomes de carbone, les ammoniums et/ou phosphoniums quartenaires pouvant également être dérivés d'hétérocycles comportant 1, 2 ou 3 atomes d'azote et/ou de phosphore, ou un cation alcalin tel que Li⁺, Na⁺ ou K⁺ [formule (9b)].

On peut aussi utiliser les phosphites représentées par la formule générale (10), pour laquelle le cation Y⁺ peut être un cation alcalin tel que Li⁺, Na⁺ ou K⁺ [formule (10b)], ou un cation organique tel qu'un ammonium quaternaire ou un phosphonium quaternaire de formule générale NR¹R²R³R⁴ et PR¹R²R³R⁴, où R¹, R², R³ et R⁴, identiques ou différents, représentent chacun l'hydrogène, un groupement hydrocarboné, aliphatique (saturé ou insaturé) ou aromatique, comprenant de 1 à 12 atomes de carbone, les ammoniums et/ou phosphoniums quartenaires pouvant également être dérivés d'hétérocycles comportant 1, 2 ou 3 atomes d'azote et/ou de phosphore.

A titre d'exemples d'ammoniums ou de phosphoniums quartenaires que l'on peut rencontrer dans les formules (9) et (10), on peut citer le tétrabutylammonium, comme dans la formule (9a) ou la formule (10a), le tétrabutylphosphonium, le N-butylpyridinium, l'éthylpyridinium, le butyl-3 méthyl-1 imidazolium, le diéthylpyrazolium et le triméthylphénylammonium.

On peut enfin utiliser les phosphites représentées par la formule générale (11), dans laquelle l'anion X est un anion faiblement coordinant. A titre d'exemples d'anions faiblement coordinants, on citera le tétrafluoroborate ou l'hexafluorophosphate, comme dans la formule (11a), le tétrachloroaluminate, l'hexafluoroantimonate, les anions carboxylates tels que l'acétate ou le trifluoroacétate, le trifluorosulfonate, les anions N(CF₃SO₂)₂⁻ et C(CF₃SO₂)₃⁻, ainsi que l'anion tétraphénylborate et les anions tétraphénylborates dont le noyaux aromatiques sont substitués.

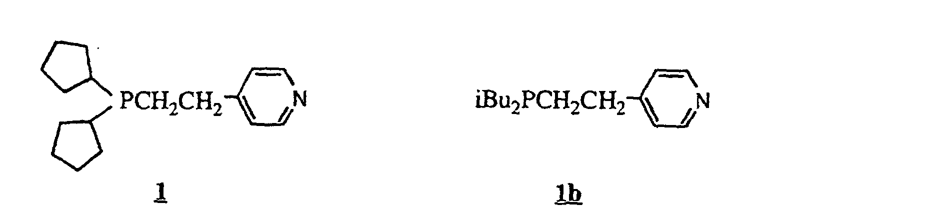

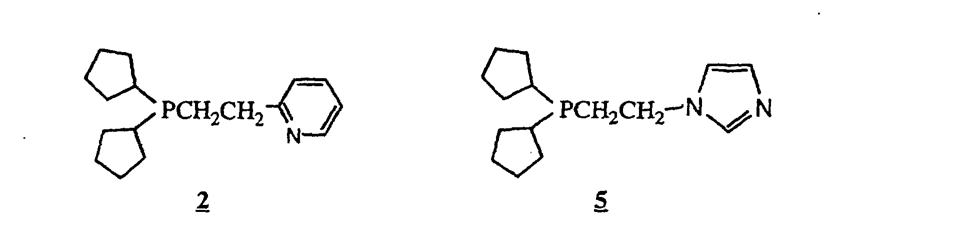

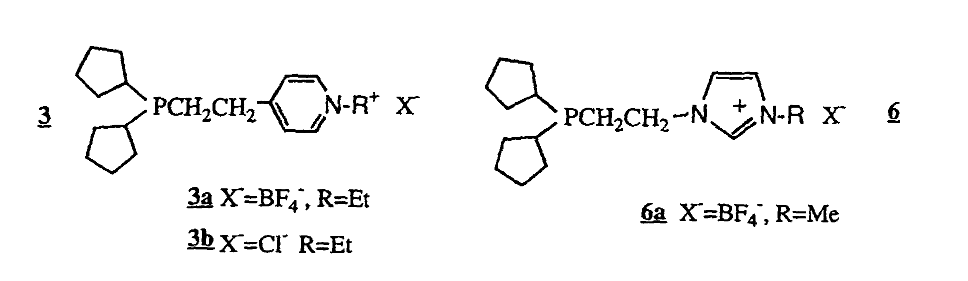

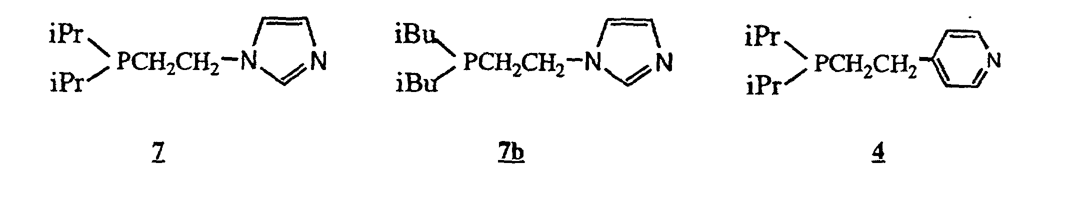

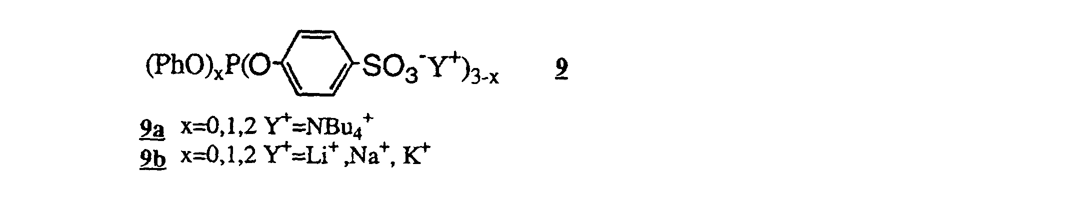

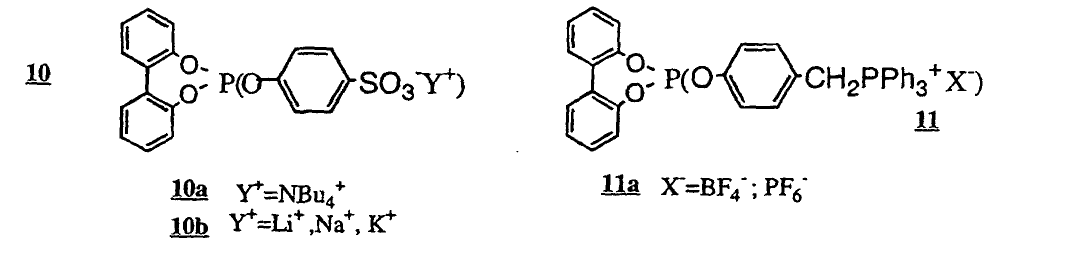

A titre d'exemples de composés du nickel utilisables dans la constitution des compositions catalytiques de l'invention, on peut citer les complexes [NiCl₂, 1.5P (dicyclopentyléthyl-2 pyridine-4)]₂, [NiCl₂,2P (dicyclopentyléthyl-2 N-éthylpyridinium tétrafluoroborate)], [Ni₂Cl₄, (dicyclopentylphosphinoéthyl-2 N-éthylpyridinium tétrafluoroborate)₃, 1,5CH₂Cl₂], NiCl₂,2 pyridine en mélange avec au moins un équivalent de phosphine tertiaire fonctionnalisée ou de phosphite fonctionnalisée, le chlorure de nickel en mélange avec au moins un équivalent de dicyclopentylphosphinoéthyl-2 pyridine-4, l'acétate de nickel en mélange avec au moins un équivalent de dicyclopentylphosphinoéthyl-2 pyridine-4, l'octoate (éthyl-2 hexanoate) de nickel en mélange avec au moins un équivalent de dicyclopentylphosphinoéthyl-2 pyridine-4 et le chlorure de π-allyl-nickel dicyclopentylphosphinoéthyl-2 pyridine-4.

Les composés entrant dans la composition catalytique selon l'invention peuvent être mélangés dans un ordre quelconque. Le mélange peut se faire par une simple mise en contact suivie d'une agitation jusqu'à formation d'un liquide homogène. Ce mélange peut être fait en dehors du réacteur de dimérisation ou d'oligomérisation ou de préférence dans ce réacteur.

Les oléfines susceptibles d'être dimérisées, codimérisées ou oligomérisées par les compositions catalytiques selon l'invention sont plus particulièrement l'éthylène, le propylène, les n-butènes et les n-pentènes, seules ou en mélange (codimérisation), pures ou diluées par un alcane, telles qu'on les trouve dans des « coupes » issues des procédés de raffinage du pétrole, comme le craquage catalytique ou le craquage a la vapeur.

La réaction catalytique de dimérisation ou d'oligomérisation des oléfines peut être conduite en système fermé, en système semi-ouvert ou en continu, avec un ou plusieurs étages de réaction. Une vigoureuse agitation doit assurer un bon contact entre le ou les réactifs et le mélange catalytique. La température de réaction peut être de -40 à +70 °C, de préférence de -20 à +50 °C. On peut opérer au-dessus ou en dessous de la température de fusion du milieu, l'état solide dispersé n'étant pas une limitation au bon déroulement de la réaction. La chaleur engendrée par la réaction peut être éliminée par tous les moyens connus de l'homme du métier. La pression peut aller de la pression atmosphérique à 20 MPa, de préférence de la pression atmosphérique à 5 MPa. Les produits de la réaction et le ou les réactifs qui n'ont pas réagi sont séparés du système catalytique par simple décantation, puis fractionnés.

Les exemples suivants illustrent l'invention sans en limiter la portée.

### EXEMPLE 1 : Préparation du solvant ionique

On a mélangé à température ambiante 17,5 g (0,1 mole) de chlorure de butyl-1 méthyl-3 imidazolium, 16,3 g (0,122 mole) de chlorure d'aluminium sublimé, 1.6 g (0,0126 mole) de dichloroéthylaluminium. On a obtenu ainsi un liquide.

### EXEMPLE 2 : Préparation du complexe [NiCl₂,1.5P (dicyclopentyléthyl-2 pyridine-4)]₂.

Dans un tube de Schlenk maintenu sous atmosphère d'argon, on introduit 2,37 g de NiCl₂,6H₂O et 10 mL de méthanol absolu. Après dissolution du sel de nickel, on ajoute 20 mL de pentane. On agite les 2 phases et on ajoute, 5,33 g de la phosphine tertiaire de formule (1) (20 mmoles). Après 2 heures d'agitation, on filtre l'insoluble rouge. On obtient 5,82g. L'analyse élémentaire correspond au complexe de formule [NiCl₂,1.5P (dicyclopentyléthyl-2 pyridine-4)]2 (M = 1085 g ; 10,7 % en poids de Ni).

### EXEMPLE 3 : Quaternisation de la pyridine du complexe décrit dans l'Exemple 2.

Dans un tube de Schlenk, on place 3,72 g du complexe décrit dans l'Exemple 2 et on ajoute du dichlorométhane. On ajoute ensuite, goutte à goutte, une solution d'oxonium de tétrafluoroborate dans le dichlorométhane (2,14 g de Et₃O⁺BF₄⁻). On laisse agiter 4 heures, temps au bout duquel on obtient une solution rouge. On évapore le solvant et on ajoute 20 mL d'éther. On filtre le solide cristallin rouge obtenu. On obtient 4,56g. L'analyse élémentaire correspond au complexe de formule: Ni₂Cl₄(P-N⁺Et BF₄⁻)₃, 1,5CH₂Cl₂, où P-N est le ligand de formule (1).

### EXEMPLE 4 : Dimérisation du propylène

Un réacteur en verre, muni d'une sonde de mesure de température, d'un barreau aimanté dans l'étage du bas (de volume 20 ml) pour assurer une bonne agitation et d'une double enveloppe permettant la circulation d'un liquide de réfrigération, a été purgé d'air et d'humidité et maintenu à la pression atmosphérique de propylène à 99 % de pureté. On y a introduit 0,03 mmole du complexe préparé dans l'Exemple 2 (0,06 mmole de Ni), puis on a abaissé la température à 10 °C et injecté à l'aide d'une seringue 5 mL de la composition liquide préparée ci-dessus (Exemple 1) et 7 mL d'heptane. On a mis l'agitation en route et on a observé immédiatement une absorption de propylène. Quand l'étage supérieur non agité a été plein de liquide, on a soutiré la plus grande partie de la phase hydrocarbonée. On a arrêté la réaction au bout de 7 heures (5 soutirages). A ce moment, on avait produit 175 kg de produits par gramme de Ni. L'analyse des différentes fractions a montré qu'elles étaient composées à 77 % de dimères. La composition des dimères qui était pratiquement identique dans toutes les fractions comportait 67 % de diméthyl-2,3 butènes, et 29 % de méthyl-pentènes le reste étant des n-hexènes.

### EXEMPLE 5 : Dimérisation du propylène

On a opéré comme dans l'Exemple 4 à ceci près qu'on a utilisé le sel fondu préparé à cet effet, et qu'on a introduit 0,05 mmole d'octoate (éthyl-2 hexanoate) de nickel et de 0,5 mmole de la dicyclopentylphosphinoéthyl-2 pyridine-4. La réaction a duré 7 heures et 15 minutes, temps au bout duquel on a soutiré 5 fractions et produit 220 kg de produits par gramme de Ni. La sélectivité en dimères est de 78 %. La sélectivité en diméthyl-2,3-butènes est de 66 % dans la première fraction et de 63 % dans la dernière.

### EXEMPLE 6 : Dimérisation du propylène

On a opéré comme dans l'Exemple 4 à ceci près qu'on a utilisé le sel fondu préparé à cet effet, et qu'on a introduit 45 mg du complexe préparé dans l'Exemple 3. La réaction a duré 7 heures et 15 minutes, temps au bout duquel on a soutiré 5 fractions et produit 117 kg de produits par gramme de Ni. La sélectivité en dimères est de 74-79 %. La sélectivité en diméthyl-2,3-butènes est de 65 % et elle reste constante dans les différentes fractions.

### EXEMPLE 7 (comparatif) : Dimérisation du propylène

On a opéré comme dans l'Exemple 4 à ceci près qu'on a utilisé le sel fondu préparé comme dans l'Exemple 1 mais en introduisant 0,05 mmole de complexe NiCl₂,2P (cyclohexyl)₃. On a laissé la réaction pendant 8 heures et 30 minutes, temps au bout duquel on a soutiré 10 fractions. On a produit 137 kg de produits par gramme de Ni, avec une sélectivité de 83 % en dimères. La sélectivité en diméthyl-2,3 butènes est de 70 % dans la première fraction ; elle chute à 35 % dans la troisième et à 10 % dans la sixième. Elle est de 6 % dans la dixième.

### EXEMPLE 8 : Dimérisation du butène

On a utilisé le sel fondu préparé dans l'Exemple 1. On a opéré comme dans l'Exemple 4 à ceci près qu'on a utilisé du butène-1 à la place du propylène. On a introduit dans l'étage inférieur du réacteur en verre 0,115 mmole (0,23 mmole de Ni = 13,5 mg de Ni) du complexe préparé dans l'Exemple 2, puis on a abaissé la température à 10 °C et on a injecté sous atmosphère de butène 5 mL de sel et 20 mL d'heptane. On a mis l'agitation en route et on a observé une absorption de butène. Quand l'étage supérieur non agité a été plein de liquide, on a soutiré la plus grande partie de la phase hydrocarbonée. On a arrêté la réaction au bout de 21 heures (28 soutirages). A ce moment, on a consommé 1708 g de butène. On avait produit 76 kg de produits par gramme de Ni. L'analyse des différentes fractions a montré qu'elles étaient composées à 80 % de dimères. L'ensemble des fractions organiques est traité par de l'acide nitrique à 10 %. On trouve 2,25 mg de Ni dans l'acide nitrique (dosage par fluorescence X). Une proportion de 19 % en poids du nickel (calculé par rapport au nickel introduit) a donc été extraite avec les produits au bout de 21 heures de réaction.

### EXEMPLE 9 : Préparation du ligand de formule (9a)

Dans un ballon tricol on introduit 26,1 g (65,58 mmole) de tétrabutylammonium d'hydroxy-4 benzène sulfonate et 100 mL de toluène. On chauffe à 140 °C et on ajoute en 1 heure 6.82 g (21.8 mmole) de triphénylphosphite et 0,385 g de trioctylamine. On laisse encore 1 heure à 140°C puis on met sous vide (10⁻⁶ mm Hg) pendant 6 heures à 110 °C. Le produit obtenu est analysé par RMN ³¹P. Il est constitué d'un mélange de 3 phosphites correspondant à x égal à 0, 1 et 2.

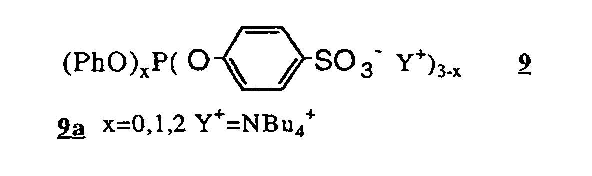

### EXEMPLE 10 : Dimérisation du butène

On a utilisé le sel fondu préparé dans l'Exemple 1. On a opéré comme dans l'Exemple 8 à ceci près qu'on a utilisé le complexe NiCl₂,2 pyridine (0,2 mmole ; 11,8 mg Ni) comme précurseur du catalyseur auquel on a ajouté 5 équivalents (670 mg) par rapport au nickel de la phosphite de formule (9a), préparé comme décrit dans l'Exemple 9. On a arrêté la réaction au bout de 43,5 heures (22 soutirages). A ce moment, on a consommé 1428 g de butène. On avait produit 73 kg de produits par gramme de Ni. L'analyse des différentes fractions a montré qu'elles étaient composées à 97-99 % de dimères. L'ensemble des fractions organiques est traité par de l'acide nitrique à 10 %. On trouve 1 mg de Ni dans l'acide nitrique (dosage par fluorescence X). Une proportion de 8,5 % en poids du nickel (calculé par rapport au nickel introduit) a donc été extraite avec les produits au bout de 43,5 heures de réaction.

### EXEMPLE 11 : Dimérisation du butène

On a opéré comme dans l'Exemple 10 à ceci près qu'on a ajouté 1 équivalent (134 mg) de la phosphite de formule (9a) par rapport au complexe NiCl₂, 2 pyridine. On a arrêté la réaction au bout de 35 heures (12 soutirages). A ce moment, on a consommé 2102g de butène. On avait produit 107 kg de produits par gramme de Ni. L'analyse des différentes fractions a montré qu'elles étaient composées à 95-97 % de dimères. L'ensemble des fractions organiques est traité par de l'acide nitrique à 10 %. On trouve 1,4 mg de Ni dans l'acide nitrique (dosage par fluorescence X). Une proportion de 12 % en poids du nickel (calculé par rapport au nickel introduit) a donc été extrait avec les produits au bout de 35 heures de réaction.

### EXEMPLE 12 (comparatif) : Dimérisation du butène

On a utilisé le sel fondu préparé dans l'Exemple 1. On a opéré comme dans l'Exemple 7 à ceci près qu'on a utilisé le complexe NiCl₂,2P(cyclohexyl)₃ (0,2 mmole de Ni ; 11,8 mg de Ni) comme précurseur de catalyseur et 40 mL d'heptane. On a arrêté la réaction au bout de 14,8 heures (9 soutirages). On observe une nette diminution de la consommation de butène. A ce moment, on a consommé 815 g de butène. On avait produit 84 kg de produits par gramme de Ni. L'analyse des différentes fractions a montré qu'elles étaient composées à 90-94 % de dimères. L'ensemble des fractions organiques est traité par de l'acide nitrique à 10 %. On trouve 6,2 mg de Ni dans l'acide nitrique (dosage par fluorescence X). Une proportion de 52 % en poids du nickel (calculé par rapport au nickel introduit) a donc été extraite avec les produits au bout de 14,8 heures de réaction.

## Revendications

1. Composition catalytique **caractérisée en ce qu'**elle comprend au moins un composé du nickel mélangé ou complexé avec au moins une phosphine tertiaire portant un groupe fonctionnel ou une phosphite portant un groupe fonctionnel, dissous au moins en partie dans un milieu non aqueux à caractère ionique résultant de la mise en contact d'au moins un halogénure d'aluminium (Produit B) avec au moins un halogénure d'ammonium quaternaire et/ou au moins un halogénure de phosphonium quaternaire (Produit A).

2. Composition catalytique selon la revendication 1 **caractérisée en ce que** le composé du nickel est choisi parmi le chlorure, le bromure, le sulfate, les carboxylates, les phénates et l'acétylacétonate.

3. Composition catalytique selon la revendication 1 ou 2 **caractérisée en ce que** la phosphine tertiaire portant un groupe fonctionnel répond à l'une des formules générales PR'₁R₂',R₃' ou R'₁R'₂P-R'-PR'₁R'₂, dans lesquelles R₁' R'₂ et R'₃, identiques ou différents, sont des radicaux alkyles, cycloalkyles, aryles ou aralkyles comportant de 1 à 10 atomes de carbone dont l'un au moins porte un groupe fonctionnel choisi parmi les groupes amines, amines cycliques, hétérocycles azotés, esters, acides, alcools, ammoniums quaternaires, phosphoniums quaternaires, sulfoniums, sulfonates et phosphonates et R' est un reste bivalent aliphatique de 1 à 6 atomes de carbone.

4. Composition catalytique selon la revendication 3 **caractérisée en ce que** la phosphine tertiaire fonctionnelle est choisie parmi les phosphines contenant des substituants pyridine ou imidazole et leurs dérivés de quaternisation à substituants pyridinium ou imidazolium.

5. Composition catalytique selon la revendication 4 **caractérisée en ce que** la phosphine tertiaire fonctionnelle portant un substituant pyridine ou imidazole est choisie parmi la dicyclopentyl-phosphinoéthyl-2-pyridine-4, la dicyclopentyl-phosphinoéthyl-2-pyridine-2, la diisobutyl-phosphinoéthyl-2-pyridine-4, la diisopropyl-phosphinoéthyl-2 pyridine-4, la dicyclopentyl-phosphinoéthyl-2 N-imidazole, la diisopropyl-phosphinoéthyl-2 N-imidazole et la diisobutyl-phosphinoéthyl-2 N-imidazole.

6. Composition catalytique selon la revendication 4 **caractérisée en ce que** la phosphine tertiaire fonctionnelle portant un substituant pyridinium ou imidazolium est un dérivé de quaternisation répondant à l'une des formules : 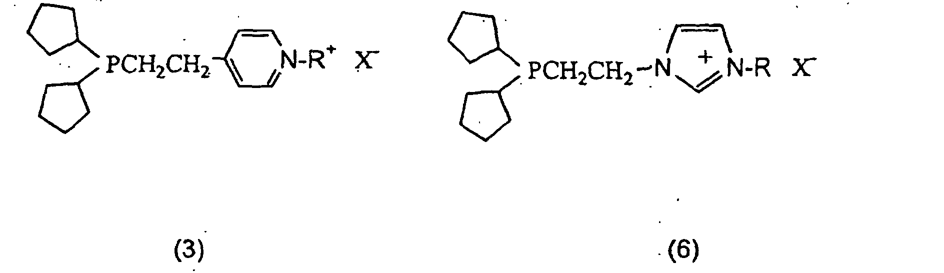 dans lesquelles R est un groupe alkyle comportant 1 à 10 atomes de carbone et X est un anion faiblement coordinant.

7. Composition catalytique selon la revendication 6 **caractérisée en ce que** l'anion faiblement coordinant est choisi parmi le tétrafluoroborate, l'hexa fluorophosphate, le tétrachloroaluminate, l'hexafluoroantimonate, les anions carboxylates, le trifluorosulfonate, et les anions N(CF₃SO₂)₂⁻ et C(CF₃SO₂)₃⁻.

8. Composition catalytique selon la revendication 7 **caractérisée en ce que** l'anion carboxylate est choisi parmi l'acétate et le trifluoroacétate.

9. Composition catalytique selon la revendication 7 ou 8 **caractérisée en ce que** la phosphine tertiaire fonctionnelle portant un substituant pyridinium ou imidazolium est choisie parmi le tétrafluoroborate de dicyclopentyl-phosphinoéthyl-2 N-éthylpyridinium, le chlorure de dicyclopentyl-phosphinoéthyl-2 N-éthylpyridinium et le tétrafluoroborate de dicyclopentyl-phosphinoéthyl-2 méthyl-1 imidazolium.

10. Composition catalytique selon la revendication 1 ou 2 **caractérisée en ce que** la phosphite fonctionnelle répond à l'une des formules générale:
P(OR"₁)(OR"₂)(OR"₃) et (-O-R"₅-O-)P(OR"₂),
dans lesquelles R"₁, R"₂, R"₃ et R"₅, identiques ou différents, sont des radicaux aryles ou aralkyles dont l'un au moins porte un groupe fonctionnel choisi parmi les amines, les amines cycliques, les hétérocycles azotés, les esters, les acides, les alcools, les ammoniums quaternaires, les phosphoniums quaternaires, les sulfoniums, les sulfonates et les phosphonates.

11. Composition catalytique selon la revendication 10 **caractérisée en ce que** la phosphite fonctionnelle répond à la formule générale 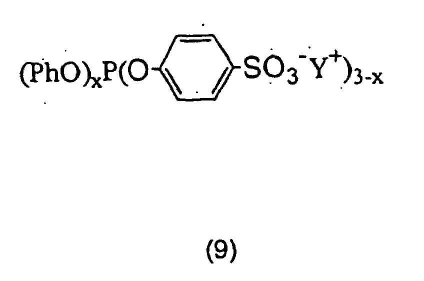 avec x= 0 à 2 dans laquelle le cation Y
- est choisi parmi le sodium, le lithium ou le potassium et les cations ammoniums quaternaires et phosphoniums quaternaires de formules générales :
N⁺R¹R²R³R⁴ et p⁺R¹R²R³R⁴
dans lesquelles R¹, R², R³ et R⁴, identiques ou différents, représentant chacun l'hydrogène, un groupement hydrocarboné, aliphatique, saturé ou insaturé, ou aromatique, comprenant de 1 à 12 atomes de carbone ;
- ou dérive d'un hétérocycle comportant 1, 2 ou 3 atomes d'azote et/ou phosphore

12. Composition catalytique selon la revendication 10 **caractérisée en ce que** la phosphite répond à la formule générale 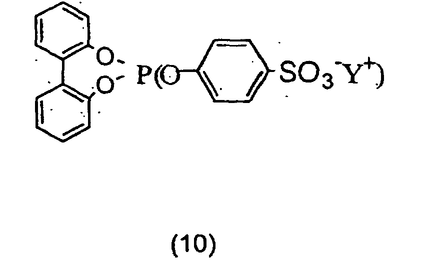 dans laquelle le cation Y
- est choisi parmi le sodium, le lithium ou le potassium et les cations ammoniums quaternaires et phosphoniums quaternaires de formules générales :
N⁺R¹R²R³R⁴ et P⁺R¹R²R³R⁴
dans lesquelles R¹, R², R³ et R⁴, identiques ou différents, représentant chacun l'hydrogène, un groupement hydrocarboné, aliphatique, saturé ou insaturé, ou aromatique, comprenant de 1 à 12 atomes de carbone ;
- ou dérive d'un hétérocycle comportant 1, 2 ou 3 atomes d'azote et/ou phosphore.
- ou dérive d'un hétérocycle comportant 1, 2 ou 3 atomes d'azote et/ou phosphore.

13. Composition catalytique selon la revendication 11 ou 12 **caractérisée en ce que** l'ammonium ou le phosphonium quartenaire est choisi parmi le tétrabutylammonium, le tétrabutylphosphonium, le N-butylpyridinium, l'éthylpyridinium, le butyl-3 méthyl-1 imidazolium, le diéthylpyrazolium et le triméthylphényl ammonium.

14. Composition catalytique selon la revendication 10 **caractérisée en ce que** la phosphité répond à la formule générale 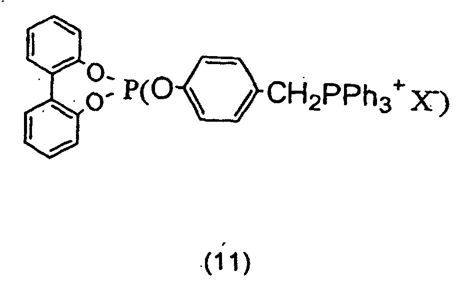 dans laquelle l'anion X est un anion faiblement coordinant.

15. Composition catalytique selon la revendication 14 **caractérisée en ce que** l'anion faiblement coordinant est choisi parmi le tétrafluoroborate, l'hexafluorophosphate, le tétrachloroaluminate, l'hexafluoroantimonate, les anions carboxylates tels que l'acétate et le trifluoroacétate, le trifluorosulfonate, les anions N(CF₃SO₂)₂⁻ et C(CF₃SO₂)₃⁻, l'anion tétraphénylborate et les anions tétraphénylborates dont le noyaux aromatiques sont substitués.

16. Composition catalytique selon les revendications 10 à 15 **caractérisée en ce que** la phosphite tertiaire portant un groupe fonctionnel est choisie parmi les phosphites décrites par les formules 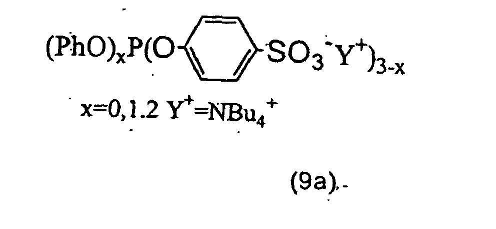 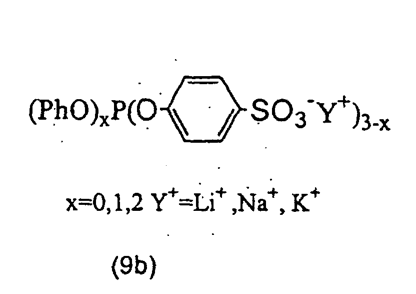 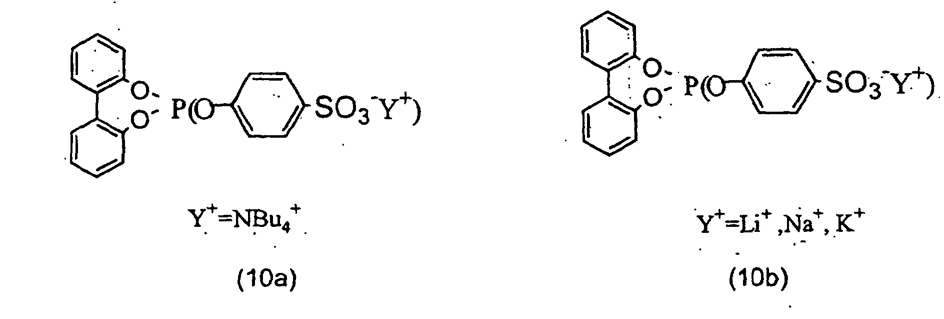 et

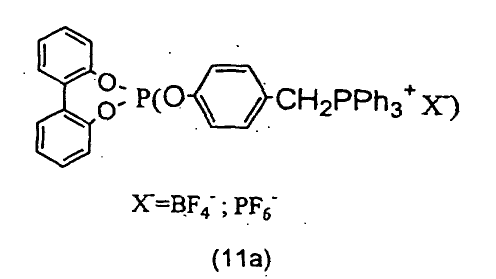

17. Composition catalytique selon l'une des revendications 1 à 16 **caractérisée en ce que** le composé du nickel mélangé ou complexé avec au moins une phosphine tertiaire portant un groupe fonctionnel est choisi parmi les complexes :
- [NiCl₂,1.5P (dicyclopentyléthyl-2 pyridine-4)]₂ ;
- [NiCl₂,2P (dicyclopentyléthyl-2 N éthylpyridinium tétrafluoroborate)]₂ ;
- [Ni₂Cl₄, (dicyclopentyl phosphinoéthyl-2 N - éthylpyridinium tétrafluoro-. borate)₃, 1,5CH₂Cl₂];
- NiCl₂,2 pyridine en mélange avec au moins un équivalent de phosphine tertiaire fonctionnalisée ou de phosphite fonctionnalisée ;
- le chlorure de nickel en mélange avec au moins un équivalent de dicyclopentylphosphinoéthyl-2 pyridine-4 ;
- l'acétate de nickel en mélange avec au moins un équivalent de dicyclopentylphosphinoéthyl-2 pyridine-4 ;
- le chlorure de π-allyl-nickel dicyclopentylphosphinoéthyl-2 pyridine-4

18. Composition catalytique selon l'une des revendications 1 à 17 **caractérisée en ce que** l'halogénure d'ammonium quaternaire ou l'halogénure de phosphonium quaternaire utilisable comme Produit A répond
- à l'une des formulés générales
NR¹R²R³R⁴X, à l'exception de NH₄X,PR¹R²R³R⁴X, R¹R²N=CR³R⁴X ou R¹R²P=CR³R⁴X,
dans lesquelles X représente Cl ou Br et R¹, R², R³ et R⁴, identiques ou différents, représentent chacun l'hydrogène ou un reste hydrocarbyle de 1 à 12 atomes de carbone,
- ou encore à l'une des formules générales : 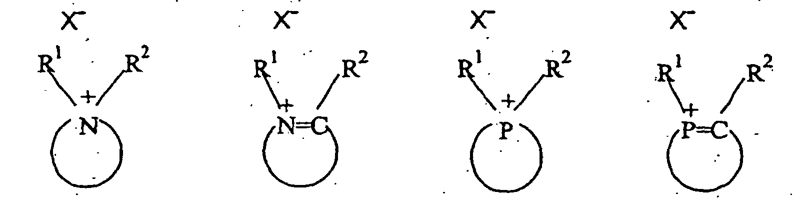
dans lesquelles les hétérocycles azotés ou phosphorés comportant 1, 2 ou 3 atomes d'azote et/ou de phosphore sont constitués de 4 à 10 atomes et X, R1 et R2 sont définis comme précédemment.

19. Composition catalytique selon la revendication 18 **caractérisée en ce que** l'halogénure d'ammonium quaternaire ou l'halogénure de phosphonium quaternaire est le chlorure de tétrabutylphosphonium, le chlorure de N-butyl pyridinium, le bromure d'éthylpyridinium, le chlorure de butyl-3 méthyl-1 imidezolium, le chlorure de diéthylpyrazolium, le chlorhydrate de pyridinium ou le chlorure de triméthylphénylammonium, le chlorure d'éthyl-1 méthyl-3 imidazolium.

20. Composition catalytique selon l'une des revendications 1 à 19 **caractérisée en ce que** l'halogénure d'aluminium utilisé comme Produit B est le chlorure ou le bromure d'aluminium

21. Composition catalytique selon l'une des revendications 1 à 20 **caractérisée en ce que** les Produits A et B sont mis en oeuvre dans un rapport molaire A:B de 1:0.5 à 1:3.

22. Composition catalytique selon l'une des revendications 1 à 21 **caractérisée en ce que** le milieu non aqueux à caractère ionique comprend en outre un Produit C, consistant en au moins un composé organométallique de l'aluminium.

23. Composition catalytique selon la revendication 22 **caractérisée en ce que** le composé organométallique de l'aluminium utilisé comme Produit C répond à la formule générale AIRₓX₃₋ₓ, dans laquelle R est un reste alkyle, linéaire ou ramifié, comportant de 2 à 8 atomes de carbone, X étant le chlore ou le brome et x a une valeur égale à 1, 2 ou 3.

24. Composition catalytique selon la revendication 22 ou 23 **caractérisée en ce que** le Produit C est le sesquichlorure. d'isobutylaluminium, le sesquichlorure d'éthylaluminium, le dichloroisobutylaluminium, le dichloroéthylaluminium ou le chlorodiéthylaluminium.

25. Composition catalytique selon l'une des revendications 22 à 24 **caractérisés en ce que** le Produit C est mis en oeuvre dans un rapport molaire avec le Produit B au plus égal à 100:1

26. Procédé de dimérisation, de codimérisation ou d'oligomérisation d'au moins une oléfine, **caractérisé en ce que** ladite oléfine est mise en contact avec une composition catalytique selon l'une des revendications 1 à 25.

27. Procédé selon la revendication 26 **caractérisé en ce que** la réaction de dimérisation, de codimérisation ou d'oligomérisation des oléfines est conduite en système fermé, en système semi-ouvert ou en continu, avec un ou plusieurs étages de réaction, sous agitation et à une température de -40 à +70 °C.

28. Procédé selon l'une des revendications 26 et 27 **caractérisé en ce que** les oléfines sont l'éthylène, le propylène, les n-butènes et les n-pentènes, seuls ou en mélange, purs ou dilués par un alcane.

29. Procédé selon l'une des revendications 26 à 28 **caractérisé en ce que** les oléfines sont contenues dans des « coupes » issues des procédés de raffinage du pétrole

## Claims

1. A catalytic composition **characterised in that** it comprises at least one nickel compound mixed or complexed with at least one tertiary phosphine or a phosphite carrying a functional group, at least partly dissolved in a non aqueous medium with an ionic nature resulting from bringing at least one aluminium halide (product B) into contact with at least one quaternary ammonium halide and/or at least one quaternary phosphonium halide (product A)

2. A catalytic composition according to claim 1 **characterized in that** the nickel compound is selected from the chloride, bromide, sulphate, carboxylates, phenates and acetylacetonate.

3. A catalytic composition according to claim 1 or claim 2 **characterized in that** the tertiary phosphine carrying a functional group has one of the general formulae PR'₁R'₂R'₃ or R'₁R'₂P-R'-PR'₁R'₂, where R'₁, R'₂ and R'₃, which may be identical or different, are alkyl, cycloalkyl, aryl or aralkyl radicals containing 1 to 10 carbon atoms at least one of which carries a functional group selected from an amine, a cyclic amine, a nitrogen-containing heterocycle, an ester, an acid, an alcohol, a quaternary ammonium, a quaternary phosphonium, a sulphonium, a sulphonate and a phosphonate group and R' is a divalent aliphatic residue containing 1 to 6 carbon atoms.

4. A catalytic composition according to claim 3 **characterized in that** the functional tertiary phosphine is selected from phosphines containing pyridine or imidazole substituents and their quatemisation derivatives with pyridinium or imidazolium substituents.

5. A catalytic composition according to claim 4 **characterized in that** the functional tertiary phosphine carrying a pyridine or imidazole substituent is selected from 2-dicyclopentylphosphinoethyl-4-pyridine, 2-dicyclopentylphosphinoethyl-2-pyridine, 2-diisobutylphosphinoethyl-4-pyridine, 2-diisopropylphosphinoethyl-4-pyridine, 2-dicyclopentylphosphinoethyl-N-imidazole, 2-diisopropylphosphinoethyl-N-imidazole and 2-diisobutylphosphinoethyl-N-imidazole.

6. A catalytic composition according to claim 4 **characterized in that** the functional tertiary phosphine carrying a pyridinium or imidazolium substituent is a quaternisation derivative with one of formulae 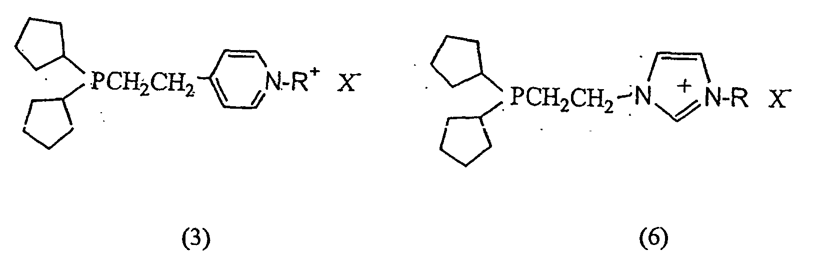 where R is an alkyl group containing 1 to 10 carbon atoms and X is a weakly co-ordinating anion.

7. A catalytic composition according to claim 6 **characterized in that** the weakly co-ordinating anion is selected from tetrafluoroborate, hexafluorophosphate, tetrachloroaluminate, hexafluoroantimonate, carboxylate anions, trifluorosulphonate, and the N(CF₃SO₂)₂- and C(CF₃SO₂)₃⁻ anions.

8. A catalytic composition according to claim 7 **characterized in that** the carboxylate anion is selected among acetate and trifluoroacetate.

9. A catalytic composition according to claim 7 or claim 8 **characterized in that** the functional tertiary phosphine carrying a pyridinium or imidazolium substituent is selected from 2-dicyclopentylphosphinoethyl-N-ethyl pyridinium tetrafluoroborate, 2-dicyclopentylphosphinoethyl-N-ethyl pyridinium chloride and 2-dicyclopentylphosphinoethyl-1-methylimidazolium tetrafluoroborate.

10. A catalytic composition according to claim 1 or claim 2 **characterized in that** the functional phosphite has one of the general formulae:
P(OR"₁) (OR"₂) (OR"₃) or (-O-R"₅-O-)P(OR"₂);
where R"₁, R"₂, R"₃ and R"s, which may be identical or different, are aryl or aralkyl radicals wherein at least one carries a functional group such as an amine, a cyclic amine, a nitrogen-containing heterocycle, an ester, an acid, an alcohol, a quaternary ammonium, a quaternary phosphonium, a sulphonium, a sulphonate or a phosphonate.

11. A catalytic composition according to claim 10 **characterized in that** the functional phosphite has general formula 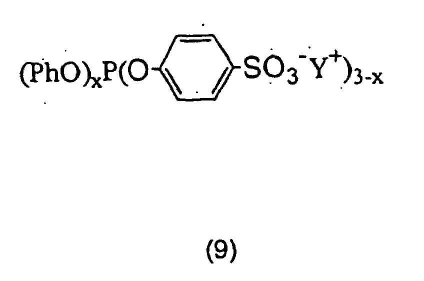 where x=0 to 2, in which cation Y:
• is selected from sodium, lithium or potassium and the quaternary ammonium and quaternary phosphonium cations have general formulae:
N⁺R¹R²R³R⁴ and P⁺R¹R²R³R⁴
where R¹, R², R³ and R⁴, which may be identical or different, each represent hydrogen, a saturated or unsaturated aliphatic or an aromatic hydrocarbon group containing 1 to 12 carbon atoms; or
• is derived from a heterocycle containing 1, 2 or 3 nitrogen and/or phosphorus atoms.

12. A catalytic composition according to claim 10 **characterized in that** the phosphite has general formula 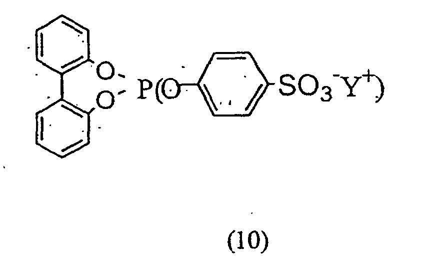 where cation Y
• is selected from sodium, lithium or potassium and quaternary ammonium and quaternary phosphonium cations with general formulae:
N⁺R¹R²R³R⁴ and P⁺R¹R²R³R⁴
where R¹, R², R³ and R⁴, which may be identical or different, each represent hydrogen, a saturated or unsaturated aliphatic or an aromatic hydrocarbon group containing 1 to 12 carbon atoms; or
• a heterocyclic derivative containing 1, 2 or 3 nitrogen and/or phosphorus atoms.

13. A catalytic composition according to claim 11 or claim 12 **characterized in that** the quaternary ammonium or phosphonium is selected from tetrabutylammonium, tetrabutylphosphonium, N-butylpyridinium, ethylpyridinium, 3-butyl-1-methyl imidazolium, diethylpyrazolium and trimethylphenyl ammonium.

14. A catalytic composition according to claim 10 **characterized in that** the phosphite has general formula 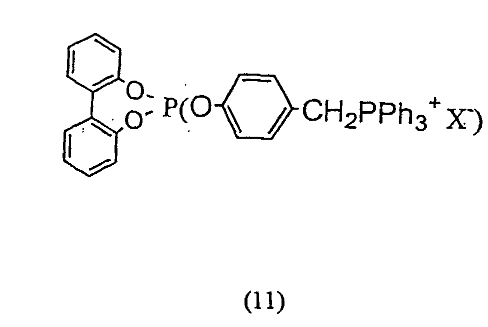 where anion X is a weakly co-ordinating anion.

15. A catalytic composition according to claim 14 **characterized in that** the weakly co-ordinating anion is selected from tetrafluoroborate, hexafluorophosphate, tetrachloroaluminate, hexafluoroantimonate, carboxylate anions such as acetate, trifluoroacetate, trifluorosulphonate, the N(CF₃SO₂)₂- and C(CF₃SO₂)₃⁻ anions, the tetraphenylborate anion and tetraphenylborate anions wherein the aromatic rings are substituted.

16. A catalytic composition according to claims 10 to 15 **characterized in that** the tertiary phosphite carrying a functional group is selected from phosphites described by formulae 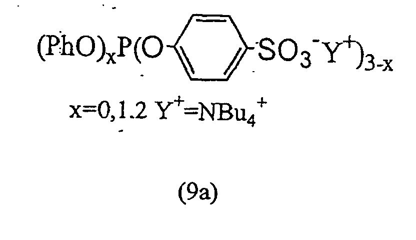 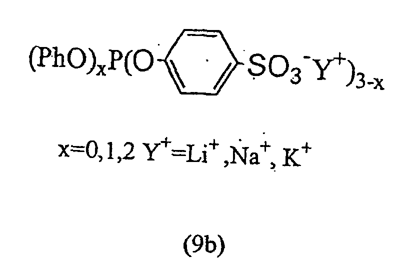 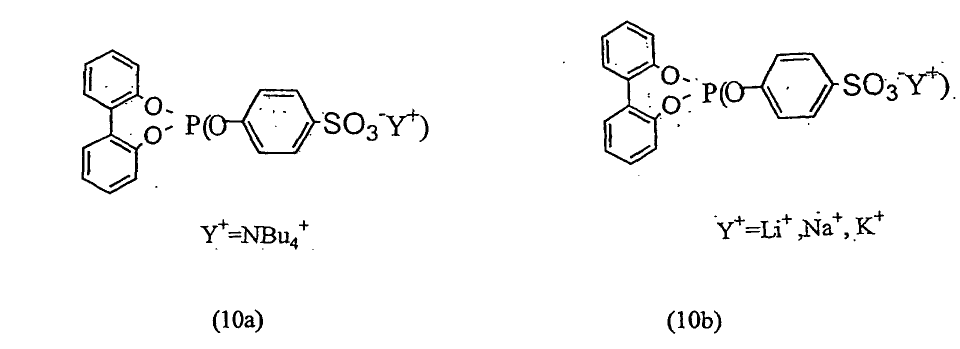 and

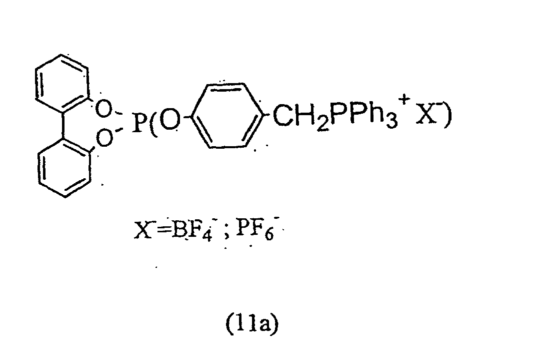

17. A catalytic composition according to any one of claims 1 to 16 **characterized in that** the nickel compound mixed or complexed with at least one tertiary phosphine carrying a functional group is selected from the following complexes:
• [NiCl₂, 1.5P(2-dicyclopentylethyl-4-pyridine)]₂;
• [NiCl₂, 2P(2-dicyclopentylethyl-N-ethyl pyridinium tetrafluoroborate)]₂;
• [Ni₂Cl₄, (2 - dicyclopentylphosphinoethyl - N- ethylpyridinium tetrafluoroborate)₃, 1.5CH₂Cl₂];
• NiCl₂, 2 pyridine mixed with at least one equivalent of functionalised tertiary phosphine or functionalised phosphite;
• nickel chloride mixed with at least one equivalent of 2-dicyclopentylphosphinoethyl-4-pyridine;
• nickel acetate mixed with at least one equivalent of 2-dicyclopentylphosphinoethyl-4-pyridine;
• nickel (2-ethyl hexanoate) octoate mixed with at least one equivalent of 2-dicyclopentylphosphinoethyl-4-pyridine;and
• 2-dicyclopentylphosphinoethyl-4-pyridine π-allyl nickel chloride.

18. A catalytic composition according to any one of claims 1 to 17 **characterized in that** the quaternary ammonium halide or quaternary phosphonium halide used as product A satisfies:
• one of general formulae: NR¹k²R³R⁴X with the exception of NH₄X, PR¹R²R³R⁴X, R¹R²N=CR³R⁴X or R¹R²P=CR³R⁴X,
where X represents Cl or Br and R¹, R², R³ and R⁴, which may be identical or different, each represent hydrogen or a hydrocarbyl residue containing 1 to 12 carbon atoms;
• or one of the following general formulae: 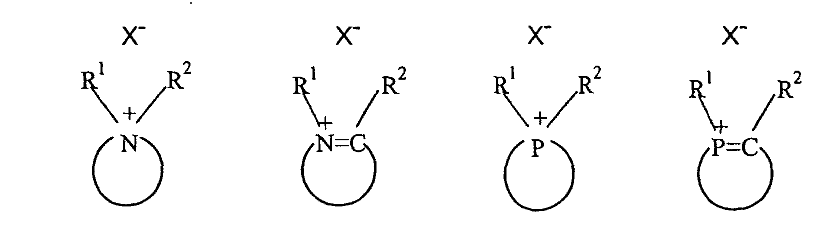
where the nitrogen-containing or phosphorus-containing heterocycles containing 1, 2 or 3 nitrogen and/or phosphorus atoms are constituted by 4 to 10 atoms and X, R¹ and R² are defined as above.

19. A catalytic composition according to claim 18 **characterized in that** the quaternary ammonium halide or quaternary phosphonium halide is tetrabutyl phosphonium chloride, N-butyl pyridinium chloride, ethylpyridinium bromide, 3-butyl-1-methyl imidazolium chloride, diethylpyrazolium chloride, pyridinium hydrochloride, trimethylphenylammonium chloride or 1-ethyl-3-methyl imidazolium chloride.

20. A catalytic composition according to any one of claims 1 to 19 **characterized in that** the aluminium halide used as product B is aluminium chloride or bromide.

21. A catalytic composition according to any one of claims 1 to 20 **characterized in that** products A and B are used in an A:B mole ratio of 1:0.5 to 1:3.

22. A catalytic composition according to any one of claims 1 to 21 **characterized in that** the nonaqueous medium with an ionic nature also comprises a product C, consisting of at least one organometallic aluminium compound.

23. A catalytic composition according to claim 22 **characterized in that** the organometallic aluminium compound used as optional product C of the invention has general formula AlRₓX₃₋ₓ where R is a linear or branched alkyl residue containing 2 to 8 carbon atoms, X is chlorine or bromine and the value of x is 1, 2 or 3.

24. A catalytic composition according to claim 21 or claim 23 **characterized in that** product C is isobutylaluminium sesquichloride, ethylaluminium sesquichloride, dichloroisobutylaluminium, dichloroethylaluminium, or chlorodiethylaluminium.

25. A catalytic composition according to any one of claims 1 to 24, **characterized in that** product C is used in a mole ratio of at most 1:100 with product B.

26. A process for dimerising co dimerising or oligomerising at least one olefin, **characterized in that** said olefin is brought into contact with a catalytic composition according to any one of claims 1 to 25.

27. A process according to claim 26 **characterized in that** the dimerisation, co-dimerisation or oligomerisation reaction is carried out in a closed system, in a semi-open system or in a continuous system, with one or more reaction stages, with agitation and at a temperature of -40°C to +70°C.

28. A process according to claim 26 or claim 27 **characterized in that** the olefins are ethylene, propylene, n-butenes and n-pentenes, used alone or as a mixture, pure or diluted by an alkane.

29. A process according to one of claims 26 to 28 **characterized in that** the olefins are contained in "cuts" from oil refining processes.

## Patentansprüche

1. Katalytische Zusammensetzung, **dadurch gekennzeichnet, dass** sie wenigstens eine Nickeverbindung, vermischt oder komplexiert mit wenigstens einem tertiären Phosphin umfasst, das eine funktionelle Gruppe trägt, oder einem Phosphit, das eine funktionelle Gruppe trägt, wenigstens teilweise gelöst in einem nichtwässrigen Medium mit ionischem Charakter, resultierend aus dem kontaktieren wenigstens eines Aluminiumhalogenids (Produkt B), mit wenigstens einem quaternären Ammoniumhalogenid und/oder wenigstens einem quarternären Phosphoniumhalogenid (Produkt A).

2. Katalytische Zusammensetzung nach Anspruch 1, **dadurch gekennzeichnet, dass** die Nickelverbindung gewählt wird unter Chlorid, Bromid, Sulfat, Carboxylaten, Phenaten und Acethylacetonat.

3. Katalytische Zusammensetzung nach Anspruch 1 oder 2, **dadurch gekennzeichnet, dass** das tertiäre Phosphin, das eine funktionelle Gruppe trägt, einer der allgemeinen Formeln PR'₁R'₂R'₃ oder R'₁R'₂P-R'-PR'₁R'₂, entspricht, in denen R'1, R'2 und R'3 identisch oder verschieden Alkyl-, Cycloalkyl-, Aryloder Aralkylreste sind, die 1 bis 10 Kohlenstoffatome umfassen, wovon wenigstens einer eine funktionelle Gruppe trägt, die gewählt ist unter den Gruppen Amine, Cycloamine, Stickstoffheterozyklen, Ester, Säuren, Alkohole, quaternäre Amonium, quaternäre Phosphonium, Sulfonium, Sulfonate und Phosphonate und R' ein bivalenter aliphatischer Rest mit 1 bis 6 Kohlenstoffatomen ist.

4. Katalytische Zusammensetzung nach Anspruch 3, **dadurch gekennzeichnet, dass** das funktionelle, tertiäre Phosphin gewählt wird unter den Phosphinen, die Pyridin-, oder Imidazolsubstituenten enthalten und deren Quartärnisierungsderivaten mit Pyridinium- oder Imidazoliumsubstituenten.

5. Katalytische Zusammensetzung nach Anspruch 4, **dadurch gekennzeichnet, dass** das funktionelle oder tertiäre Phosphin, das einen Pyridin- oder Imidazolsubstituenten trägt, gewählt wird unter Dicyclopentyl-2-Phosphinoethyl-4-Pyridin, Dicyclopentyl-2-Phosphinoethyl-2-Pyridin, Diisobutyl-2-Phosphinoethyl-4-Pyridin, Diisopropyl-2-Phosphinoethyl-4-Pyridin, Dicyclopentyl-2-Phosphinoethyl-N-imidazol, Diisopropyl-2-Phosphinoethyl-N-Imidazol und Diisobutyl-2-Phosphinoethyl-N-lmidazol.

6. Katalytische Zusammensetzung nach Anspruch 4, **dadurch gekennzeichnet, dass** das funktionelle tertiäre Phosphin, das einen Pyridinium- oder Imidazolsubstituenten trägt, ein Quaternisierungsderivat ist, das einer der Formeln entspricht. 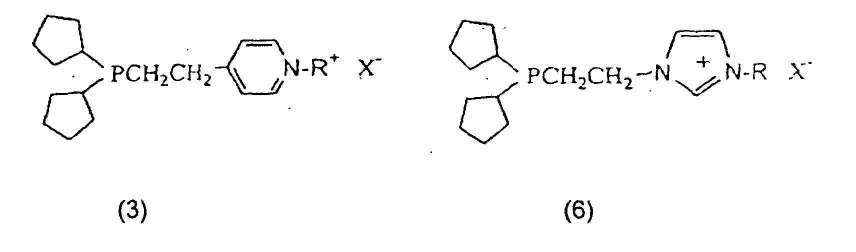 in denen R eine Alkylgruppe ist, die 1 bis 10 Kohlenstoffatome umfasst und X ein schwach koordinierendes Anion ist.

7. Katalytische Zusammensetzung nach Anspruch 6, **dadurch gekennzeichnet, dass** das schwach koordinierende Anion gewählt ist unter Tetrafluorborat, Hexafluorphosphat, Tetrachloraluminat, Hexafluorantimonat, den Carboxylatanionen, Trifluorsulfonat und den Anionen, N(CF₃SO₂)₂⁻ und C(CF₃SO₂)₃⁻.

8. Katalytische Zusammensetzung nach Anspruch 7, **dadurch gekennzeichnet, dass** das Carboxylatanion gewählt wird unter Acetat und Trifluoracetat.

9. Katalytische Zusammensetzung nach Anspruch 7 oder 8, **dadurch gekennzeichnet, dass** das funktionelle tertiäre Phosphin, das einen Pyridinium- oder Imidazoliumsubstituenten trägt gewählt wird unter Tetrafluorborat von Dicyclopentyl-2-Phosphinoethyl-N-Ethylpyridinium, Chlorid von Dicyclopentyl-2-Phosphinoethyl-N-Ethylpirridinium und Tetrafluorborat von Dicyclopentyl-2-Phosphinoethyt-1-Methylimidazolium.

10. Katalytische Zusammensetzung nach Anspruch 1 oder 2, **dadurch gekennzeichnet, dass** das funktionelle Phosphit eine der allgemeinen Formeln entspricht:
P(OR"₁)(OR"₂)(OR"₃) und (-O-R"₅-O-)P(OR" ₂)
in denen R"₁, R"₂, R"₃ und R"₅ identisch oder verschieden Aryl- oder Aralkylreste sind, von denen wenigstens einer eine funktionelle Gruppe trägt, gewählt unter den Aminen, Cycloaminen, Stickstoffheterozyklen, Estern, Säuren, Alkoholen, quaternären Ammonium, quaternären Phosphonium, Sulfonium, Sulfonaten und Phosphonaten.

11. Katalytische Zusammensetzung nach Anspruch 10, **dadurch gekennzeichnet, dass** das funktionelle Phosphit der allgemeinen Formel entspricht 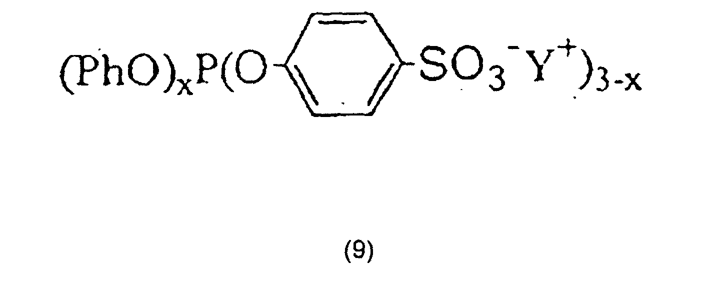 mit x=0 bis 2 in welcher das Kation Y
- gewählt ist unter Natrium, Lithium oder Kalium und den quaternären Ammoniumkationen und quaternären Phosphoniumkationen mit allgemeinen Formeln
N⁺R¹R²R³R⁴ und P⁺R¹R²R³R⁴
in denen R¹, R², R³ und R⁴ identisch oder verschieden jedes Wasserstoff, eine Kohlenwasserstoffgruppe, aliphatisch, gesättigt oder ungesättigt oder aromatisch darstellen, die 1 bis 12 Kohlenstoffatome umfasst;
- oder abgeleitet von einem Heterozyklus, der 1,2 oder 3 Stickstoffund/oder Phosphoratome umfasst.

12. Katalytische Zusammensetzung nach Anspruch 10, **dadurch gekennzeichnet, dass** das Phosphit der allgemeinen Formel entspricht 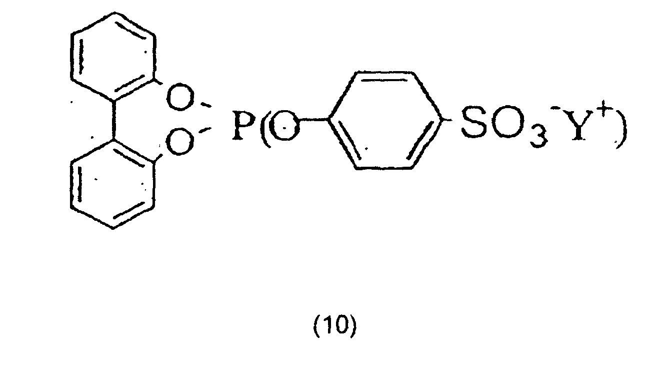 in der das Kation Y
- gewählt ist unter Natrium, Lithium oder Kalium und den quaternären Ammoniumkationen und quaternären Phosphoniumkationen mit allgemeinen Formeln
N⁺R¹R²R³R⁴ und P⁺R¹R²R³R⁴
in denen R¹, R², R³ und R⁴ identisch oder verschieden jedes Wasserstoff, eine Kohlenwasserstoffgruppe, aliphatisch, gesättigt oder ungesättigt oder aromatisch darstellen, die 1 bis 12 Kohlenstoffatome umfasst;
- oder abgeleitet von einem Heterozyklus, der 1, 2 oder 3 Stickstoffund/oder Phosphoratome umfasst,
- oder abgeleitet von einem Heterozyklus, der 1, 2 oder 3 Stickstoffund/oder Phosphoratome umfasst.

13. Katalytische Zusammensetzung nach Anspruch 11 oder 12, **dadurch gekennzeichnet, dass** das quaternäre Amonium oder Phosphonium gewählt ist unter Tetrabutylammonium, Tetrabutylphosphonium, N-Butylpyridinium, Ethylpyridinium, 3-Butyl-1-Methylimidazolium, Diethylpyrazolium und Trimethylphenylammonium.

14. Katalytische Zusammensetzung nach Anspruch 10, **dadurch gekennzeichnet, dass** das der Phosphit der allgemeinen Formel 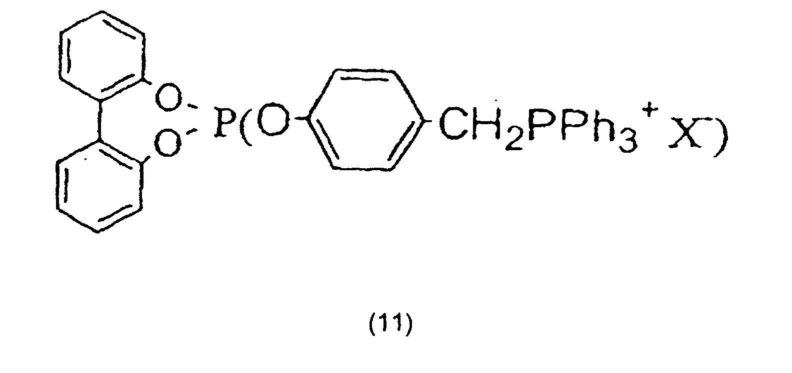 entspricht, in welcher das Anion X ein schwach koordinierendes Anion ist.

15. Katalytische Zusammensetzung, **dadurch gekennzeichnet, dass** das schwach koordinierende Anion gewählt ist unter Tetrafluorborat, Hexafluorphosphat, Tetrachloraluminat, Hexafluorantimonat, den Carboxylatanionen wie Acetat und Trifluoracetat, Trifluorsulfonat, den Anionen N(CF₃SO₂)₂⁻ und C(CF₃SO₂)₃⁻ , dem Tetraphenylboratanion und den Tetraphenylboratanionen, deren aromatische Ringe substituiert sind.

16. Katalytische Zusammensetzung nach den Ansprüchen 10 bis 15, **dadurch gekennzeichnet, dass** das tertiäre Phosphit, das eine funktionelle Gruppe trägt, die gewählt ist unter den Phosphiten, die durch die Formeln beschrieben werden 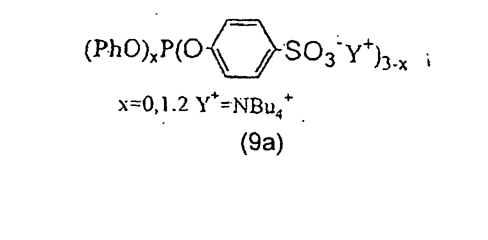 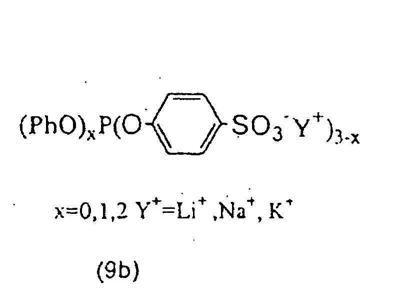 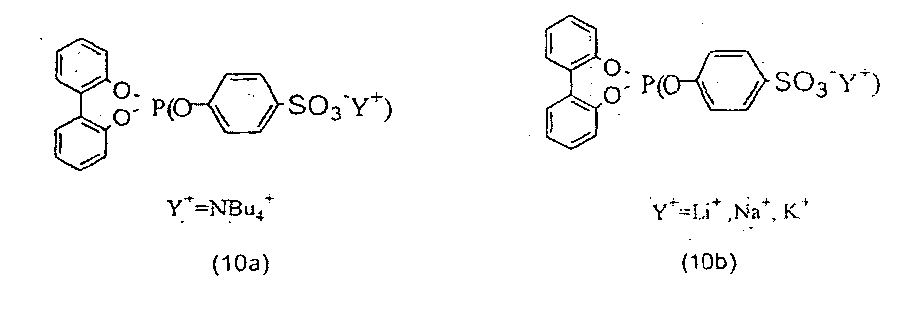 und

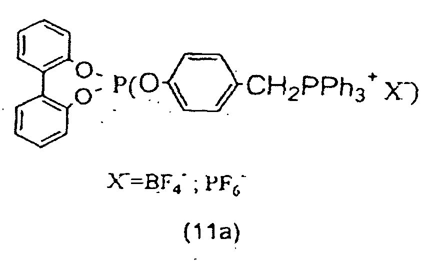

17. Katalytische Zusammensetzung nach einem der Ansprüche 1 bis 16, **dadurch gekennzeichnet, dass** die Nickelverbindung, vermischt oder komplexiert mit wenigstens einem tertiären Phosphin, das eine funktionelle Gruppe trägt, gewählt wird unter den Komplexen
- [NiCl₂, 1.5P (Dicyclopentylethyl-2-(4-Pyridin))]₂;
- [NiCl₂,2P (Dicyclopentylethyl-2-(N-Ethylpiridinium)-Tetrafluorborat)]₂;
- [Ni₂Cl₄, (Dicyclopentyl-2-Phospinethyl-N-Ethylpyridinum-Tetrafluorborat)₃ 1,5 CH₂Cl₂]
- NiCl₂, 2-Pyridin im Gemisch mit wenigstens einem Äquivalent funktionalisiertem tertiären Phosphin oder funktionalisiertem Phosphit;
- Nickelchlorid im Gemisch mit wenigstens einem Äquivalent Dicyclopentyl-2-Phosphinoethyl-4-Pyridin;
- Nickelacetat im Gemisch mit wenigstens einem Äquivalent von Dicyclopentyl-2-Phosphinoethyl-4-Pyridin;
- Chlorid von π-Allyl-Nickel Dicyclochiopentyl-2-Phosphinethyl-4-Pyridin.

18. Katalytische Zusammensetzung nach einem der Ansprüche 1 bis 17, **dadurch gekennzeichnet, dass** das quaternäre Ammoniumhalogenid oder quarternäre Phosphoniumhalogenid, das als Produkt A verwendbar ist, entspricht
- einer der allgemeinen Formeln:
NR¹R²R³R⁴X, mit Ausnahme von NH₄X, PR¹R²R³R⁴X , R¹R²N=CR³R⁴X oder R¹R²P=CR³R⁴X,
in denen X Cl oder Br darstellt und R¹, R², R³ und R⁴ identisch oder verschieden jedes Wasserstoff oder ein Kohlenwasserstoffrest mit 1 bis 12 Kohlenstoffatomen darstellen,
oder auch einer der allgemeinen Formeln 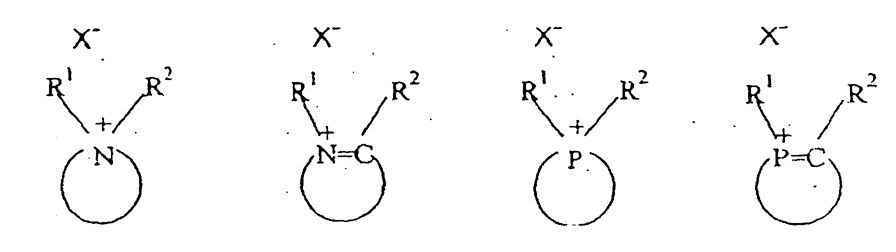 in denen die stickstoff- oder phosphorhaltigen Heterozyklen, die 1, 2 oder 3 Stickstoff- und/oder Phosphoratome umfassen, bestehen aus 4 bis 10 Atomen und X, R¹ und R², wie oben definiert sind.

19. Katalytische Zusammensetzung nach Anspruch 18, **dadurch gekennzeichnet, dass** das quarternäre Ammoniumhalogenid oder quarternäre Phosphoniumhalogenid das Chlorid von Tetrabutylphosphonium, das Chlorid von N-Butylpyridinium, das Bromid von Ethylpyridinium, das Chlorid von 3-Butyl-1-Methylimidazolium, das Chlorid von Diethylpyrazolium, das Chlorhydrat von Pyridinium oder das Chlorid von Trimethylphenylammonium, das Chlorid von 1-Ethyl-3-Methylimidazolium ist.

20. Katalytische Zusammensetzung nach einem der Ansprüche 1 bis 19, **dadurch gekennzeichnet, dass** das als Produkt B verwendete Aluminiumhalogenid das Chlorid oder das Bromid von Aluminium ist.

21. Katalytische Zusammensetzung nach einem der Ansprüche 1 bis 20, **dadurch gekennzeichnet, dass** die Produkte A und B in einem Molverhältnis A:B von 1:0,5 bis 1:3 eingesetzt werden.

22. Katalytische Zusammensetzung nach einem der Ansprüche 1 bis 21, **dadurch gekennzeichnet, dass** das nicht wässrige Milieu mit ionischem Charakter außerdem ein Produkt C umfasst, das aus wenigstens einer organometallischen Aluminiumverbindung besteht.

23. Katalytische Zusammensetzung nach Anspruch 22, **dadurch gekennzeichnet, dass** die organometallische, als Produkt C verwendete Aluminiumverbindung der allgemeinen Formel AlRₓX₃₋ₓ entspricht, in der R ein linearer oder verzweigter Alkylrest ist, der 2 bis 8 Kohlenstoffatome umfasst, wobei X Chlor oder Brom ist und x einen Wert gleich 1, 2 oder 3 hat.

24. Katalytische Zusammensetzung nach Anspruch 22 oder 23, **dadurch gekennzeichnet, dass** das Produkt C, das Sesquichlorid von Isobutylaluminium, das Sesquichlorid von Ethylaluminium, das Dichlorisobutylaluminium, Dichlorethylaluminium oder Chlordiethylaluminium ist.

25. Katalytische Zusammensetzung nach einem der Ansprüche 22 bis 24, **dadurch gekennzeichnet, dass** das Produkt C in einem Molverhältnis mit dem Produkt B höchstens gleich 100:1 eingesetzt wird.

26. Verfahren zur Dimerisierung, Codimerisierung oder Oligomerisierung von wenigstens einem Olefin, **dadurch gekennzeichnet, dass** das Olefin mit einer katalytischen Zusammensetzung nach einem der Ansprüche 1 bis 25 kontaktiert wird.

27. Verfahren nach Anspruch 26, **dadurch gekennzeichnet, dass** die Reaktion zur Dimerisierung, Codimerisierung oder Oligomerisierung der Olefine im geschlossenen System, im halboffenen System oder kontinuierlich mit einer oder mehreren Reaktionsstufen unter Rühren bei einer Temperatur von -40 bis +70°C durchgeführt wird.

28. Verfahren nach einem der Ansprüche 26 und 27, **dadurch gekennzeichnet, dass** die Olefine Ethylen, Propylen, n-Butene und n-Pentene allein oder im Gemisch, rein oder verdünnt durch ein Alkan sind.

29. Verfahren nach einem der Ansprüche 26 bis 28, **dadurch gekennzeichnet, dass** die Olefine in den "Fraktionen" aus den Erdölraffinierungsverfahren enthalten sind.
